# EUROPEAN PATENT SPECIFICATION

(11) **EP 3 317 656 B1**
(45) Date of publication and mention of the grant of the patent: **21.08.2024**
(21) Application number: 16734648.5
(22) Date of filing: 01.07.2016
(51) Int. Cl.: G01N 29/02, G01N 29/032, C12Q 1/68, G01N 29/036, G01N 33/543

(54) **MEASUREMENT OF ANALYTE WITH AN ACOUSTIC WAVE SENSOR**
MESSUNG EINES ANALYTEN MIT EINEM SCHALLWELLENSENSOR
MESURE D'ANALYTE AVEC UN CAPTEUR D'ONDE ACOUSTIQUE

(30) Priority: 03.07.2015 GB 201511687
(43) Date of publication of application: 09.05.2018
(73) Proprietor: Foundation For Research And Technology Hellas, Crete (GR)
(72) Inventor: PAPADAKIS, George, 71305 Crete (GR); GIZELI, Electra, 71409 Crete (GR)
(74) Representative: Hindles Limited
(86) International application number: PCT/EP2016/065612
(87) International publication number: WO 2017/005663

(56) References cited:
- US-A- 4 236 893
- US-A1- 2002 023 493
- US-A1- 2005 148 065
- US-A1- 2009 170 119
- US-A1- 2010 105 079
- HIROTSUGU OGI ET AL: "Replacement-free mass-amplified sandwich assay with 180-MHz electrodeless quartz-crystal microbalance biosensor", BIOSENSORS AND BIOELECTRONICS, ELSEVIER BV, NL, vol. 26, no. 12, 23 May 2011 (2011-05-23), pages 4819 - 4822, XP028242182, ISSN: 0956-5663, [retrieved on 20110530], DOI: 10.1016/J.BIOS.2011.05.035

## Description

### Field of the invention

The invention relates to the field of measuring the presence and/or amount of an analyte using an acoustic wave sensor.

### Background to the invention

The present invention addresses the problem of measuring a small amount of an analyte, such as a nucleic acid, protein or hormone, in a sensitive, simple and cost-effective way. Measuring includes detecting the presence (versus absence) of the analyte, which is typically a binary measurement with a yes or no outcome, or making a quantitative measurement of the amount of the analyte which is present.

The enzymatic amplification of DNA with PCR, developed in the 1980's, brought az major change in the detection of genetic markers in molecular diagnostics, changing everyday clinical practice and introducing the ability to observe the molecular basis of a disease and identify specific biomarkers. Still, today, while PCR represents the ultimate in terms of sensitivity, it has significant drawbacks including complexity, sensitivity to contamination, cost and lack of portability (Rosi, N.L.; Mirkin, C. A.; Nanostructures in biodiagnostics, Chem. Rev. 105:1547-1562 (2005)).

In addition, some DNA templates are preferentially amplified within the same reaction, a phenomenon known as PCR bias (Tan D. & Lynch H.T., Principles of Molecular Diagnostics and Personalized Cancer Medicine, Wolters Kluwer Health/Lippincott Williams & Wilkins (2013)). In some settings, PCR bias can cause 10 to 30-fold differences in amplification efficiency which could result in underestimation or failure to detect mutations. A single nucleotide polymorphism can cause significant PCR bias and this is observed with both proofreading and non-proofreading polymerases. In the case of heterogeneous samples where rare mutated sequences exist amongst abundant wild-type sequences, the PCR may be unable to amplify sufficiently these rare targets. Furthermore, non-specific PCR inhibitors, including heparin, and uncharacterized components are sometimes present in samples from patients which may lead to undesired results such as mis-priming and inhibition.

Recently, advancements in the field of nano-materials have resulted in new detection platforms; the Bio-Bar-Code (BBC) approach, one of the most promising methods, has achieved ultra-high sensitivities in DNA detection in the zM concentration range (Nam, J.M. et al., Bio-Bar-Code-based DNA detection with PCR-like sensitivity, JACS, 126:5932-5933 (2004)). However, this impressive performance, similar to that obtained with PCR, does not come in a simple format; it involves cumbersome and lengthy procedures such as the use of exogenous surface-modified components and multi-step amplification and detection schemes. When BBC was applied to the detection of bacterial genomic DNA the reported limit of detection was in the fM range (Nam J-M., et al., Nanoparticle-based Bio-Bar Codes for the ultrasensitive detection of proteins, Science, 301:1884-1886 (2003)).

With regards to the detection of protein analytes, the current gold standard is the enzyme-linked immunosorbent assay (ELISA) with a detection limit in the pM range. Again, progress in nanomaterials and through the BBC approach has allowed the detection of proteins down to the aM range (Nam J-M., et al., Nanoparticle-based Bio-Bar Codes for the ultrasensitive detection of proteins, Science, 301:1884-1886 (2003)) Similarly to the DNA-BBC assay, this method involves several exogenous particles and multi-step amplification steps.

Recently, acoustic sensors have emerged as a very important platform for biophysical and clinical analysis. Interestingly, this is not accompanied by an advancement of our comprehension of the underpinning science behind acoustic wave/soft matter interaction. The widely accepted mechanisms, i.e., that acoustic waves propagating at a solid/liquid interface are sensitive to mass and solution-viscosity changes occurring at the interface, go back to pioneering works performed in the late fifties and nineties (Sauerbrey G., Zeitschrift für Physik 155 (2): 206-222(1959); Ricco A.J. & Martin S.J., Acoustic wave viscosity sensor, Appl. Phys. Lett. 50, 1474 (1987)). Mass changes are reflected in the measurement of the acoustic velocity, i.e., frequency (F) or phase (Ph) of the wave and it is now well documented both theoretically and experimentally that ΔF and ΔPh are analogous to the amount of elastic mass deposited on the device surface (Mitsakakis, K. et al., Quantitative determination of protein-Mw with acoustic sensor; specific vs non-specific binding, Analyst, 139:3918- 3925 (2014)). For this reason clinical applications exploiting acoustic wave sensors are based on the measurement of this parameter (i.e. velocity etc.) to quantify proteins or antibodies in the nM concentration range (Mitsakakis, K.; Gizeli, E.; Detection of multiple cardiac markers with an integrated acoustic platform for cardiovascular risk assessment, Anal. Chim. Act. 699:1(2011); Lee J. et al.,Sensitive and simultaneous detection of cardiac markers in human serum using SAW immunosensor, Anal. Chem. 83:8629 (2011)). Recently, much improved detection limits (pM) were reported by using a sandwich immunoassay in which the subsequent catalyzed deposition of gold (Au) onto Au-nanoparticles led to an enhancement of the acoustic signal (Lee J., et al., Sensitive and reproducible detection of cardiac troponin I in human plasma using a surface acoustic wave immunosensor, Sens. Act. B., 178:19-25 (2013)). WO 2008/145130 (Atonomics A/S) is similar to the BBC method in terms of using gold deposition for signal enhancement; in terms of the acoustic detection, the deposition of extra mass results in a much higher phase response.

It is also known to detect labelled analyte from a measurement of changes in acoustic velocity, i.e., frequency (F) or phase (Ph) from, for example: US 2002/023493 (Miyake Jun et al.), US 2009/170119 (Lee Hun Joo et al.), US 2010/105079 (Warthoe Peter et al.), US 4 236 893 (Rice Thomas K), and "Replacement-free mass-amplified sandwich assay with 180-MHz electrodeless quartz-crystal microbalance biosensor", Biosensors and Bioelectronics, Elsevier BV, NL., vol. 26, no. 12, 23 May 2011 (Hirotsugu Ogi et al.).

Viscosity changes occurring during the loading of pure solutions, for example, glycerol, are depicted in the energy dissipation measurement, normally expressed as dissipation (D) or amplitude (A). However, the mechanism by which acoustic energy is dissipated when biomolecules are attached to the device surface is still unclear and unexploited in clinical applications. We have previously developed a theory which attributes energy losses to hydrodynamic coupling phenomena (EP 2171083). Briefly, a drag force is produced by oscillating biomolecules (attached to the surface via a single point) in the surrounding liquid and this is energy consuming. Rigorous theoretical treatment showed that the hydrodynamic parameter of relevance was the intrinsic viscosity [η] of the bound molecule/particle which can be related to the ratio of ΔD/ΔF or ΔA/ΔPh (Tsortos, A. et al., Quantitative determination of size and shape of surface-bound DNA using an acoustic wave sensor, Biophys. J. 94: 2706-2715 (2008); Tsortos, A. et al., Shear acoustic wave biosensor for detecting DNA intrinsic viscosity and conformation:A study with QCM-D, Bios. Bioel. 24: 836-841 (2008)). This mechanism depends on the size and shape of the attached entity, something proven experimentally for DNAs of various conformations and globular proteins.

The present invention aims at providing a novel approach for detecting very low concentration of analytes in solution by using acoustic waves.

Although the invention will be discussed further with reference to the measurement of analytes using a QCM and a Love wave device, the invention may be performed using other types of liquid medium acoustic wave sensor. By a liquid medium acoustic wave sensor we mean an acoustic wave sensor which supports an acoustic wave than can propagate when the sensing surface of the acoustic wave sensor is in contact with a liquid in use.

### Summary of the invention

Within this specification and the appended claims, by the dissipative capacity of the analyte, or the label, we refer to the ratio of the change in the energy losses of an acoustic wave generated by an acoustic wave sensor to the change in the frequency or phase of the acoustic wave generated by the liquid medium acoustic wave sensor, due to the binding of the analyte or label to the device surface.

One skilled in the art will appreciate that the energy losses of an acoustic wave generated by an acoustic wave sensor may be measured by measuring the amplitude or dissipation of the acoustic wave and could reflect changes of the viscoelastic properties at the device/liquid interface. The frequency and phase, are, for example, affected by mass deposited on the sensing surface of the acoustic wave sensor. Thus, the dissipative capacity of the analyte, or the label, may for example be the ratio of the change in amplitude (A) or dissipation (D) of the acoustic wave to the change in frequency (F) or phase (Ph) of the acoustic wave, resulting from the binding of the analyte, or label, to the sensing surface of the acoustic wave sensor, for example ΔD/ΔF, the ratio of the change in dissipation to the change in frequency.

This ratio, ΔD/ΔF, or ΔD/ΔPh, is known as the acoustic ratio and, in the case of the binding of discrete non-interactive biomolecules or entities, is independent of the mass which binds to the sensing surface. In those cases where the ratio depends on surface coverage, the dissipative capacity of the bound entity is defined as the ratio obtained at low surface coverages (<10%) where it can be assumed that no lateral interactions exist.

Within this specification and the appended claims, references to proteins, nucleic acids (e.g. RNA, DNA and other polymers of nucleotides), hormones, metabolites or other biological macromolecules are intended to include both natural macromolecules and synthetic variants, such as proteins including non-proteinogenic residues, nucleic acids including non-natural bases etc. The term "protein" is not intended to imply any specific minimum number of peptide residues. The term "nucleic acid" is not intended to imply any specific minimum number of nucleotides, although nucleic acids employed in the invention typically have at least 10 nucleotides.

According to a first aspect of the present invention there is provided a method of measuring an analyte using a liquid medium acoustic wave sensor according to claim 1.

The label has a dissipative capacity which is at least 10% greater than that of the analyte. We have found that by using a label with a dissipative capacity which is at least 10% greater than that of the analyte, the sensitivity of the measurement is dramatically improved compared to measurement of a corresponding amount of unlabelled analyte. It may be that the change in the measurement of the said parameter which is related to the energy losses of an acoustic wave generated by the liquid medium acoustic wave sensor due to binding of the analyte without the label is below the detection limit of the liquid medium acoustic wave sensor, but the change in the measurement of the said parameter is above the detection limit when the label binds.

Typically the label has a dissipative capacity which is at least 20% greater than, at least 25% greater than, at least 50% greater than, at least double, at least three times, or at least four times that of the analyte.

The label comprises a label body (for example a liposome) and adherence of the label to the sensing surface thereby anchors the label body to the surface with an anchor length of 5 - 250nm. By the anchor length we refer to the maximum distance from which the label body can be spaced apart from the surface due to the connection between the label body and the surface. In practice, particularly where the anchor is flexible, the anchor will not always be fully extended and so the label body may sometimes be closer to the surface. The anchor which is thereby formed between the label body and the surface typically comprises the analyte.

The label binds specifically to the analyte.The label is adhered to the sensing surface through the analyte, thereby adhering to the analyte and the surface (when analyte is present).

A specific recognition element (the surface bound specific recognition element) may be bound to the sensing surface. The surface bound specific recognition element may bind specifically to the analyte (or may be configured to bind to an analyte binding element which binds specifically to the analyte) in use. Thus, the analyte (and the label body) may be adhered to the sensing surface through the specific recognition element bound to the surface. The surface bound specific recognition element may be a single or double stranded nucleic acid, aptamer, antibody (e.g. attached to a spacer region (e.g. long chain) bound to the surface), polymeric chain (dextran, PEG etc.) or peptide, for example.

The surface bound specific recognition element may be bound to the sensing surface through a spacer region.

A surface probe may be adhered to the sensing surface and may comprise the specific recognition element, and a said spacer region intermediate the specific recognition element and the sensing surface. Thus, the analyte (and the label body) may be adhered to the surface through the surface probe.

The spacer region may have a length of at least 5nm, at least 10 nm or at least 20 nm. The spacer region may for example comprise single or double stranded nucleic acid, an aptamer, or a polymeric chain (such as dextran or polyethylene glycol).

The analyte may be adhered to the surface through a surface probe (typically comprises said specific recognition element and spacer region) which has a length, through which the analyte (and label) adhere to the surface, of at least 5nm, at least 10 nm or at least 20 nm.

The surface bound specific recognition element may comprise a nucleic acid having a sequence which is complementary to a region of the analyte (where the analyte is a nucleic acid). The surface probe may comprise a nucleic acid and the nucleic acid may comprise the surface bound specific recognition element (which is a sequence which is complementary to a region of the analyte) and a spacer region which is intermediate the surface bound specific recognition element and the sensor surface.

Typically, the said nucleic acid has a length of 15 to 735 nucleotides, 29 to 735 nucleotides or 59 to 735 nucleotides. The spacer region of the nucleic acid may have a length of at least 10 nucleotides, or at least 15 nucleotides, or at least 29 nucleotides between the surface and the specific recognition element. This spacer region, which forms part of the anchor between the label body and the surface, may be single stranded or may be double stranded in whole or part.

A specific recognition element (the label bound specific recognition element) may be bound to the label body. The label bound specific recognition element may bind specifically to the analyte (or may be configured to bind to an analyte binding element which binds specifically to the analyte) in use. Thus, the label body may be adhered to the analyte (and the sensing surface) through the label bound specific recognition element. The label bound specific recognition element may be a single or double stranded nucleic acid, aptamer, antibody (e.g. attached to a spacer region (e.g. long chain) bound to the surface), polymeric chain (dextran, PEG etc.) or peptide, for example.

The label bound specific recognition element may be bound to the label body through a spacer region.

A label bound probe may be adhered to the label body and may comprise the label bound specific recognition element, and a said spacer region intermediate the label bound specific recognition element and the label body. Thus, the label body may be adhered to the analyte (and the sensing surface) through the label bound probe.

The label bound spacer region may have a length of at least 5nm, at least 10 nm or at least 20 nm. The label bound spacer region may for example comprise single or double stranded nucleic acid, an aptamer, or a polymeric chain (such as dextran or polyethylene glycol).

The analyte may be adhered to the label body through a label bound probe (typically comprises said label bound specific recognition element and spacer region) which has a length, through which the analyte adheres to the label body, of at least 5nm, at least 10 nm or at least 20 nm.

The label bound specific recognition element may comprise a nucleic acid having a sequence which is complementary to a region of the analyte (where the analyte is a nucleic acid). The label bound probe may comprise a nucleic acid and the nucleic acid may comprise the label bound specific recognition element (which is a sequence which is complementary to a region of the analyte) and a spacer region which is intermediate the label bound specific recognition element and the label body.

Typically, the label bound nucleic acid has a length of 15 to 735 nucleotides, 29 to 735 nucleotides or 59 to 735 nucleotides. The spacer region of the label bound nucleic acid may have a length of at least 10 nucleotides, or at least 15 nucleotides, or at least 29 nucleotides between the label body and the specific recognition element. This spacer region, which forms part of the anchor between the label body and the surface, may be single stranded or may be double stranded in whole or part.

In some embodiments, the surface bound specific recognition element comprises a nucleic acid sequence which is complementary to a first region of the analyte and the label bound specific recognition element comprises a nucleic acid sequence which is complementary to a second region of the analyte (which is typically adjacent to the first region of the analyte, with a spacing of zero to 50 nucleotides).

It may be that the analyte has a length, through which the label adheres to the surface, of 5 - 250nm (optionally a length of at least 10nm, or at least 20nm) and the said label body is adhered to the sensing surface through the analyte. In this case, the analyte anchors the label body to the sensing surface with an anchor length of 5 - 250nm. Effectively, the analyte may act as a spacer with a length of 5 - 250nm. In this case the analyte may be double stranded DNA and the method may comprise the initial step of amplifying a target nucleic acid by nucleic acid amplification, for example using the polymerase chain reduction (PRC), to obtain the analyte. This can be used to detect especially low quantities of the target nucleic acid. The analyte may therefore be a double stranded DNA molecule with a length of 15 to 735 base pairs, 29 to 735 base pairs or 59 to 735 base pairs. In this case, the analyte may adhere directly to the sensing surface. It may be that the sensing surface does not comprise a specific recognition element which specifically binds the analyte. It may be that the sensing surface comprises a specific recognition element which specifically binds the analyte and which is not a nucleic acid. It may be that the sensing surface comprises a specific recognition element which binds the analyte by covalent bonding. In some embodiments, the analyte may adhere directly to the label body. In that case, it may be that the label body does not comprise a specific recognition element which specifically binds the analyte. It may be that the label body comprises a specific recognition element which binds the analyte by covalent bonding. Where the method comprises amplifying a target nucleic acid to obtain the analyte, the step of amplifying a target nucleic acid may comprise introducing one or more nucleic acid residues comprising a binding moiety for binding the sensing surface or the label body, for example, neutravidin, biotin, cholesterol or a thiol group.

It may be that the analyte adheres to the surface before the first measurement is made. It may be that the analyte adheres to the surface after the first measurement is made but before the second measurement is made. It may be that the method comprises making a preliminary measurement before the analyte adheres to the surface and then making the first measurement after the analyte adheres to the surface. This enables a measurement to be made of the change in the parameter due to adherence of the analyte. This can be compared with the change in the parameter between the first and second measurements due to adherence of the label. In some circumstances the change due to adherence of the analyte will be undetectable but the change due to adherence of the label will be detectable. The preliminary measurement, first measurement and/or the second measurement may be measurements selected from amongst continuous measurements. The preliminary measurement, first measurement and/or the second measurement may each be a combination of multiple measurements, for example averages.

It may be that the first and second measurements (and preliminary measurement where applicable) each comprise a measurement of the said parameter relating to the energy losses of an acoustic wave generated by the liquid medium acoustic wave sensor (the first signal). This measurement (the first signal) may be a measurement of the amplitude of the acoustic wave. This measurement (the first signal) may be a measurement of the dissipation of the acoustic wave. This measurement (the first signal) may be a measurement of electrical circuit analogue parameters such as the impedance, admittance, resistance, susceptance, conductance or bandwidth parameters of the acoustic sensor.

It may be that the first and second measurements (and preliminary measurement where applicable) each comprise a measurement of a parameter relating to the energy losses of an acoustic wave generated by the liquid medium acoustic wave sensor (said first signal) and also a measurement of a parameter relating to the frequency or phase of an acoustic wave generated the liquid medium acoustic wave sensor (said second signal). The change in each of these measurements may be taken into account determine either or both the presence and amount of analyte.

The liquid medium acoustic wave sensor may be a Bulk Acoustic Wave type device, such as a Quartz Crystal Microbalance, Thickness Shear Mode resonator or Thickness Shear Bulk Acoustic Resonator (for example, High Fundamental Frequency QCM (HFF-QCM) and Thickness Shear Film Bulk Acoustic Resonator (TS-FBAR)). In this case, the second parameter will typically be a measurement of the frequency of oscillation or resonance frequency of the liquid medium acoustic wave sensor and the parameter related to the energy losses of the acoustic wave (the first signal) will typically be related to the energy dissipation or bandwidth of the wave generated by the acoustic wave sensor.

The liquid medium acoustic wave sensor may be an acoustic wave sensor which generates a shear wave; such Surface Acoustic Wave type devices can employ interdigitated transducers to generate a shear wave, such as a Love wave, Surface Skimming Bulk Wave, Acoustic Plate Mode, Bleustein-Gulyaev wave, leaky acoustic waves or Surface Transverse Wave. In this case, the parameter related to the energy losses of an acoustic wave (the first signal) will typically be a measurement of the amplitude of the surface acoustic wave which is generated and the parameter related to the velocity of the acoustic wave (the second signal) will typically be a measurement of the phase of the surface acoustic wave which is generated.

The shear acoustic wave sensor may be a non-contact, non-interdigitated-transducer based device such as a device employing an electromagnetically excited shear acoustic wave. The liquid medium acoustic wave sensor may be an acoustic wave sensor using a thin membrane to excite an acoustic wave in a configuration known as Flexural Plate Wave or Lamb wave device.

It may be that the analyte is adhered to the sensing surface before the first measurement is taken. Typically, the label will then be added and binds to the analyte, where present. Typically, the label specifically binds to the analyte, or to an analyte binding element which in turn binds, typically specifically, to the analyte.

The analyte may be dsDNA fragments produced by any type of enzymatic amplification (PCR or isothermal) or hybridization or any other enzymatic reaction. In this case, the analyte may comprise one or more binding moieties to form bonds (typically covalent bonds) with the sensing surface and/or the label body. Direct binding of the ds analyte to the sensor surface, for example through a thiol modification, may eliminate the need for a specific surface-bound recognition molecule. If the analyte is sufficiently long, it may eliminate the benefit of a separate spacer region.

The analyte binds discretely to the sensing surface through a single attachment point (for example, as discrete structures which are not also bound to each other, for example discrete molecules where the analyte is a molecule). The label binds discretely to the sensing surface, so that each label is adhered to the sensing surface through a single analyte. Nevertheless, in some embodiments, the label may bind to the sensing surface together with the analyte. For example, label and analyte may be mixed in solution so that the label binds to the analyte where present, and the bound label and analyte may then be introduced to the sensing surface. In this case, the label will typically comprise a specific recognition element for specifically binding the analyte and/or the sensing surface will typically comprise a specific recognition element for specifically binding the analyte. In some cases in may be that the preformed complex of label/analyte could bind to the surface-bound specific recognition element through the label; in this case the label may have two different specific recognition elements, one for binding the analyte and another one for binding to the surface-bound specific recognition element.

The label will be any molecule, polymeric structure, entity or complex of a combination of several molecules, polymers and entities. In all cases, the label should be characterized by a dissipative capacity, which is higher than that of the analyte. Entities could comprise any kind of bodies such as nanoparticles, quantum dots, liposomes, and dendrimers or combination of the above in more complex structures. By nanoparticles we mean any particle having a diameter of 1-500 nm. Nanoparticles may be made of any material, such as a metal (e.g. gold), polymer, silica etc. By a liposome we refer to a compartment enclosed by a lipid bilayer (for example, a phospholipid bilayer), optionally with additional components attached to its surface such as polymers, DNAs, peptides or proteins. Liposomes include both unilamellar vesicles and multilamellar vesicles. Liposomes typically have a diameter of 20 to 500nm. The label may comprise liposomes encapsulating a medium with a viscosity of greater than 1 x 10⁻² Pa.s or greater than 0.1 Pa.s at 25°C, for example glycerol. The liposomes may comprise a plurality of cross-linked molecules between lipids on either side of the lipid bilayer or even within the bilayer. The label may comprise groups of a plurality of liposomes which are joined to each other, for example by dendrimers. As discussed above, the label may comprise a body (such as a said nanoparticle, quantum dot, liposome, dendrimer) and a spacer region through which the body of the label adheres to the analyte, with a length of 5 - 250nm.

In a second aspect, the invention extends to a method of selecting a label for the detection of an analyte by a liquid medium acoustic sensor according to claim 13. Also disclosed but not claimed is a kit for detecting an analyte using a liquid medium acoustic sensor having a sensing surface, the kit comprising a surface probe adhereable to a sensing surface or adhered to a sensing surface, label bodies, label probe adhered to or adherable to the label bodies, the surface probe and the label probe each comprising specific recognition elements configured to specifically bind respective regions of the analyte, the surface probe and/or the label probe comprising a spacer region such that the label bodies can be adhered discretely to the surface through the analyte and the surface probe and label probe, so that each label adheres to the sensing surface through a single analyte, to thereby anchor the label bodies to sensing surface with an anchor length of 5-250nm, the label having a dissipative capacity which is at least 10% greater than that of the analyte.

The label probe may be a said label bound probe or may be adherable to the label body to form a said label bound probe, in which case it may comprise a label body binding element (e.g. one or more chemical groups configured to bind to the label). The label probe may have a length of at least 5nm, at least 10nm or at least 20nm. The spacer region of the label probe may have a length of at least 5nm, at least 10nm or at least 20nm. The spacer region of the label probe may for example comprise single or double stranded nucleic acid, an aptamer, or a polymeric chain (such as dextran or polyethylene glycol). The label probe may comprise a nucleic acid with a length of 15 to 735 nucleotides, 29 to 735 nucleotides or 59 to 735 nucleotides. The nucleic acid may comprise a region which is the said specific recognition element of the label probe and is complementary to a region of the analyte, and a spacer region (through which the specific recognition element adheres to the label body) which has a length of at least 10 nucleotides, at least 15 nucleotides, or at least 29 nucleotides.

The surface probe may be a surface bound probe or may be adherable to a sensing surface to form a surface bound probe, in which case it may comprise one or more surface binding elements configured to bind to a sensing surface, either directly or to a sensing surface coating. The surface probe may have a length of at least 5nm, at least 10nm or at least 20nm. The spacer region of the surface probe may have a length of at least 5nm, at least 10nm or at least 20nm. The spacer region of the surface probe may for example comprise single or double stranded nucleic acid, an aptamer, or a polymeric chain (such as dextran or polyethylene glycol). The surface probe may comprise a nucleic acid with a length of 15 to 735 nucleotides, 29 to 735 nucleotides or 59 to 735 nucleotides. The nucleic acid may comprise a region which is the said specific recognition element of the surface probe and is complementary to a region of the analyte, and a spacer region (through which the specific recognition element adheres to the sensing surface) which has a length of at least 10 nucleotides, at least 15 nucleotides, or at least 29 nucleotides.

The kit may further comprise a sensing surface of a liquid medium acoustic sensor. The kit may further comprise a liquid medium acoustic sensor. The surface probe may be adhered to the said sensing surface.

Further optional features of the second aspect of the invention correspond to those discussed above in relation to the first aspect of the invention.

### Description of the Drawings

An example embodiment of the present invention will now be illustrated with reference to the following Figures in which:
Figure 1(a) is a schematic representation of a label adhered to the sensing surface of an acoustic wave sensor through a label bound probe, an analyte, and a surface probe;
Figure 1(b) is a schematic representation of a label adhered to the sensing surface of an acoustic wave sensor through a double stranded DNA analyte;
Figure 2(a) is a schematic representation of a single stranded nucleic acid surface probe;
Figure 2(b) is a schematic representation of a nucleic acid surface probe having a double stranded spacer region;
Figure 2(c) is a schematic representation of a label having a single stranded nucleic acid label bound probe;
Figure 2(d) is a schematic representation of a label having a nucleic acid label bound probe with a double stranded spacer region;
Figures 3(a) and 3(b) are schematic representation of a label adhered to a sensing surface through a nucleic acid analyte in which the surface probe has a single stranded (3(a)) or double stranded (3(b)) spacer region;
Figures 4(a) and 4(b) are schematic representation of a label adhered to a sensing surface through a nucleic acid analyte in which the label bound probe has a single stranded (4(a)) or double stranded (4(b)) spacer region;
Figure 5 is a table of results showing dissipative capacity (ΔD/ΔF) when three different lengths of double stranded DNA molecules (analytes) are adhered to a sensing surface (column (iii)) and when three different diameters of liposomes (label) are adhered to the DNA (columns (iv), (v), (vi));
Figure 6 is a graph of the change in dissipation (ΔD) when a sample of liposome label binds to surface bound DNA for six different amounts of DNA, along with a table showing the change in frequency when the corresponding amount of DNA binds;
Figure 7 is a table showing the change in frequency (in Hertz) and dissipation when the corresponding amounts of DNA shown in Fig. 6 bind to the sensing surface, without addition of label; and
Figure 8 is a schematic diagram of various possible label structures.

### Detailed Description of an Example Embodiment

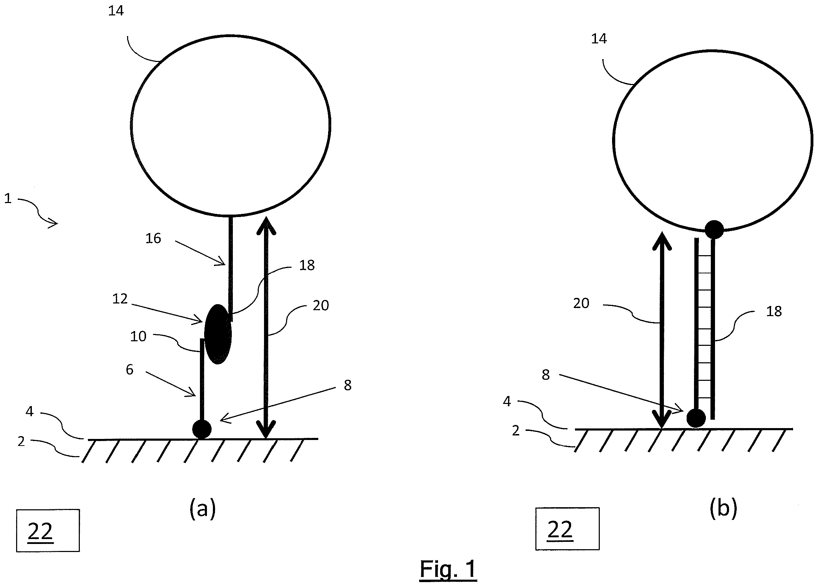

With reference to Figure 1(a), an acoustic wave device 1 has a substrate 2 having a sensing surface 4 in contact with liquid medium. A surface probe 6 is immobilised on the sensing surface through one end of the probe 8. The other end of the probe comprises a specific recognition element 10 selected to specifically bind an analyte 12. Initially the analyte is not present.

A sample which is to be assayed for the analyte is then added to the liquid medium (or the liquid medium is replaced with the sample, for example in a flow through embodiment) and a label construct is added. The label construct 14, 16 has a body (14), for example liposomes, and a label-bound probe 16 which has a specific recognition element 18 which also selectively binds the analyte. Excess label is then rinsed away.

Accordingly, the label is adhered to the sensing surface, through the analyte. Importantly, the label-bound probe, analyte and surface probe function in combination as an anchor which adheres the body of the label to the sensing surface so that the body of the label is anchored at a maximum distance (labelled 20) of 5 to 250nm from the sensing surface. In practice the anchor may be flexible and so the label may sometimes be closer to the surface. The label is selected to have a dissipative capacity which is at least 10% greater than the analyte.

The acoustic wave device has a control unit 22 which is operated to generate an acoustic wave at the liquid interface and measurements are made of (1) the energy losses of the acoustic wave and/or (2) either the frequency or phase of the acoustic wave depending on the type of acoustic wave device. For example, for a QCM device, the dissipation or the frequency of the wave, or both, are measured. Typically, measurements of energy loss and/or either frequency or phase are made continuously while the analyte is added, followed by the label, but this is not essential. In general, first measurements of these parameters should be made before the label is adhered to the surface and second measurements should be made after the label is adhered to the surface. In practice, it is helpful to make measurements before the analyte is present, after the analyte has been added but before the label is added, and again once the label is present.

As a result of the higher dissipative capacity of the label, and as a result of the spacing of the label body from the sensing surface, we have found that the change in measured parameter(s) which arise due the binding of the label are much greater than the changes which arise from binding of the analyte, enabling much lower concentration of analyte to be detected than could be detected from the change in the measured parameter(s) arising simply from the binding of the analyte.

The assay can be qualitative (determining whether analyte was detected or not), or the change in the measured parameter(s) can be compared with a calibration curve to make a quantitative measurement of analyte concentration in a sample.

In an alternative configuration, illustrated with reference to Figure 1(b) the analyte itself (12) may function as an anchor to anchor the body of the label to the sensing surface at a maximum distance of 5 to 250nm. This configuration is for example suitable where the analyte is double stranded DNA having a length of 15-800 base pairs. In this case, the analyte may be adhered to the label in solution and then introduced to the sensing surface so that labelled analyte adheres to the sensing surface. A first measurement is taken before the label and analyte adhere together to the sensing surface and a second measurement is taken after the label and analyte adhere. Alternatively, the analyte may be adhered first to the sensing surface and label added later as with the example of Figure 1(a). The analyte may for example be modified (e.g. with a cholesterol, biotin or thiol modification) to adhere to the label.

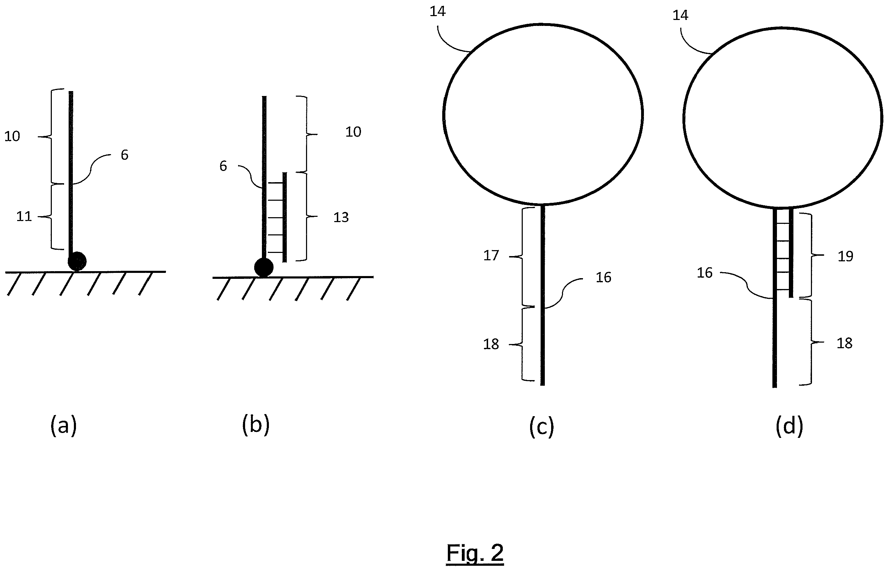

Figure 2(a) and Figure 2(b) illustrate two possible configurations of surface probe where the analyte is a single stranded nucleic acid. In the example of Figure 2(a) the surface probe 6 comprises a single stranded RNA or DNA molecule which has a specific recognition element 10 at one end, in the form of a nucleic acid sequence which is complementary to a first end of an analyte nucleic acid strand, and a spacer region 11, which is the part of the surface probe between the surface and where the complementary sequence begins. In Figure 2(b) the surface probe has a double stranded spacer region 13 and one strand extends further to form the specific recognition element 10.

Figure 2(c) and Figure 2(d) illustrate two possible configurations of the label bound probe. In the example of Figure 2(c), the label bound probe 16 comprises a single stranded RNA or DNA molecule which has a sequence which is complementary to one end of the analyte (the opposite end to the part recognised by the surface probe) which functions as a specific recognition element 18. The other end, which is adhered to the body of the label, functions as a spacer region (17). In Figure 2(d), the label-bound probe has a double stranded spacer region 19 and one strand extends beyond the double stranded portion to form the specific recognition element 18.

In the examples of Figures 2(a) through 2(d) the length of the anchor between the body of the label and the sensing surface is the length of the spacer region of the surface probe (11 or 13) plus the length of the spacer region of the label bound probe (17 or 19) plus the length of the analyte nucleic acid strand 12 between the specific recognition elements (10, 18) plus some additional length due to the chemical groups which bind the surface probe to the surface and the label bound probe to the body of the label. The length of this anchor is 5-250nm. Typically, the length of the spacer regions (11, 13, 17, 19) are in the range 50-200 nucleotides/base pairs. The analyte could potentially extend further to either side of the region which binds the specific recognition elements but any additional length does not contribute to the overall length of the anchor between the body of the label and the surface.

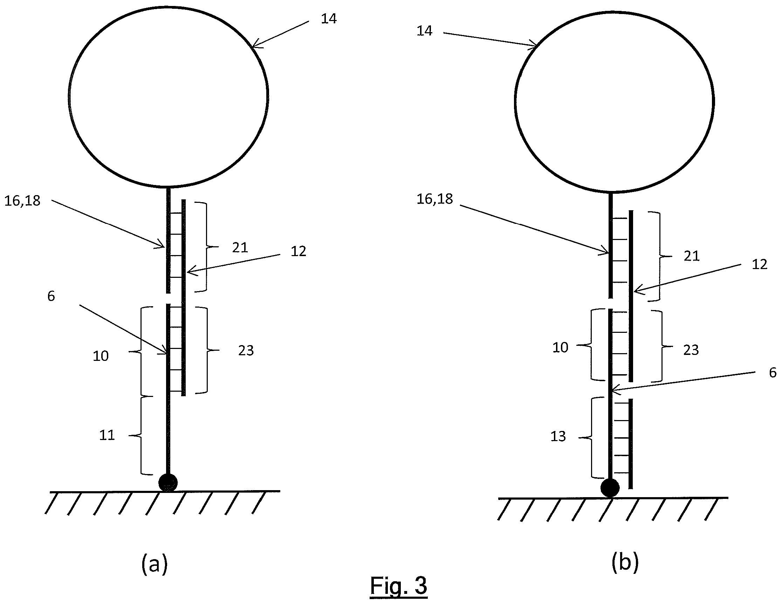

Figure 3(a) and Figure 3(b) illustrate two possible configurations for detecting either single stranded nucleic acid analytes or DNA (in which case one strand of the DNA functions as the analyte 12 which is adhered to the surface). In the embodiment of Figure 3(a), the surface probe 6 comprises a spacer region 11 which extends to a region 10 which is complementary to a surface probe binding region 23 of the analyte and which functions as the specific binding element, as per Figure 2(a). The label bound probe 16, 18 is entirely complementary to a further label binding region 21 of the analyte. In the embodiment of Figure 3(b), the surface probe comprises a double stranded spacer region 13 with one strand extending beyond the spacer region to form the specific recognition element 10, as per Figure 2(b), and the label bound probe 16, 18, is again entirely complementary to a further part of the analyte.

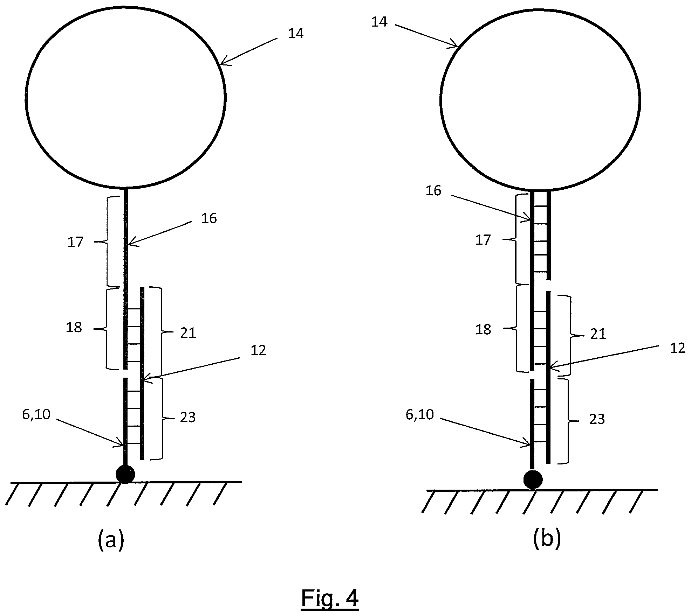

Figure 4(a) and Figure 4(b) illustrate two further possible configurations for detecting either single stranded nucleic acid analytes or DNA (in which case one strand of the DNA functions as the analyte 12 which is adhered to the surface). In these embodiments, the surface probe 6, 10 is entirely complementary to a region of the analyte and the label bound probe 16 has a specific binding region 18 which is complementary to a further region of the analyte and a single stranded (Figure 4(a)) or double stranded (Figure 4(b)) spacer region 17 or 19 respectively. In alternative configurations the spacer regions of both the surface probe and label binding probe may be double stranded, at least in part. In embodiments where the spacer regions comprise a double stranded region the strand which extends to form the specific binding region may also comprise a further part of the spacer region between the double stranded region and the specific binding region.

In the cases of Figures 3(a), 3(b), 4(a) and 4(b), the length of the anchor between the body of the label and the surface is determined by the length of the label bound probe plus the length of the surface probe plus the length of the gap between the label bound probe and surface probe spanned by the analyte, plus the length of chemical groups which bind the surface probe to the surface and the label bound probe to the body of the label. Again, the length of this anchor is 5-250nm. Typically, the length of the spacer regions (11, 13, 17, 19) are in the range 50-200 nucleotides/base pairs. The analyte could potentially extend further to either side of the region which binds the specific recognition elements but any additional length does not contribute to the overall length of the anchor between the body of the label and the surface.

In these examples, the sample containing the analyte and label have been added as discrete solutions, but one skilled in the art will appreciate that many variations can be employed. For example, the analyte and label may flow past the sensing surface. The label may also bind to the analyte before the analyte binds to the sensing surface, thereby binding the label to the sensing surface.

### Example Implementation

In an example implementation for the detection of a single stranded nucleic acid analyte the acoustic wave sensor is a Quartz Crystal Microbalance (QCM) constructed as described in the Materials and Methods section below. The sensor has a quartz crystal substrate 2 and a sensing surface formed by a surface gold layer 4 to which neutravidin is adsorbed. A 5'-biotinylated single stranded DNA molecule is used as the surface probe 6. This probe is formed through PCR or an isothermal amplification process using a suitable set of primers and introduced to the liquid medium which is in contact with the sensing surface. (Short 5'-biotinylated single stranded DNA molecules are also commercially available). The surface probe single stranded DNA adheres to the sensing surface by virtue of the specific interaction between biotin 8 and neutravidin on the sensing surface. Each DNA molecule is individually attached through the biotin which is attached to the end of the DNA molecule. One skilled in the art will appreciate that a surface probe can be immobilised on a surface layer using any of a number of alternative chemistries.

Non-specific binding of the analyte to the surface is eliminated by using standard protocols familiar to those skilled in the art, such as using blocking agents, biocompatible surfaces, PEG-modified layers etc.

The surface probe 6 has a spacer region 11 with a length of at least 10 nucleotides adjacent the surface and the other end of the single stranded DNA functions has a DNA sequence which functions which is complementary to a region of an analyte nucleic acid 18 and so functions as the specific recognition element 10.

In use, a liquid sample which contains (or which is to be assayed for the presence of) analyte is brought into contact with the sensing surface in a buffer ensuring hybridising conditions. The target analyte 12, in this example a single stranded circulating tumour DNA molecule (ctDNA), specifically binds to the surface probe by virtue of the interaction between a surface probe capturing region 19 of the analyte 12 and the specific recognition element 10 of the DNA surface probe 6. The liquid sample is rinsed off and the acoustic wave sensor is used to make a first measurement of the dissipation and frequency of the QCM before label is added.

In order to obtain the measurement the energy (which constitutes the first signal) and frequency (which constitutes the second signal) of the acoustic wave is measured on a continuous basis. The DNA which is attached to the device surface results in dissipation of the energy of the acoustic wave, measured as dissipation change. If the number of DNA molecules present in the sample and bound to the surface through the specific recognition element is very low, then the binding of the analyte will not produce a measurable signal. In this example, the DNA is present in very small amounts and its direct binding does not produce a measurable acoustic signal.

Next, a label is added in a buffer ensuring hybridizing conditions and hybridizing conditions are maintained. In this case, the label comprises liposomes 14 having single stranded DNA molecules bound thereto by virtue of a 5'-cholesterol modification and functioning as the label bound probe 16. At the end of the label bound probe which has not bound to the surface there is a sequence 18 which is complementary to the label binding region 21 of the target analyte. DNA molecules which are suitable for the label binding probe can be produced by PCR or an isothermal amplification method using a suitable primer. The label binding region 21 of the analyte is typically adjacent the surface probe binding region 23. Hence, the liposomes 14 are specifically adhered to the analyte 18 (in this case, ctDNA) and thereby to the sensing surface 4. The label bound probe and surface probe are selected so that the sum of their length plus the length of any gap between the label binding region 21 and the surface probe binding region 23 of the analyte, has a length of 5 to 250nm).

A second measurement is taken of the dissipation and frequency of acoustic waves, once the label has been added and has bound to the surface through analyte (where present). The change in dissipation (ΔD) and frequency (ΔF) of the acoustic wave between the first and second measurements is calculated. A statistically significant change in either dissipation or frequency is indicative that analyte is present and the amount of the change dissipation and/or frequency can be compared against a calibration curve to give a quantitative measurement of the amount of analyte. As the liposomes have a much greater dissipation capacity than single stranded DNA, the change in the dissipation and frequency due to the binding of the liposomes adhered to the surface is much greater than that due to the binding of the analyte single stranded DNA; in some cases, as explained above, the binding of the analyte on its own prior to binding of the label may give zero frequency or dissipation change. Hence the detection limit for the analyte is greatly improved in comparison to that which is possible with a measurement of the change in dissipation or frequency when the analyte alone binds to the sensing surface.

### Experimental Results

In order to demonstrate the ability of highly dissipative labels to enhance the acoustic signal and detection level of analytes, the effect on dissipation and frequency due to the binding of DNA and liposomes was compared. Double stranded DNA closely models the DNA structures formed using the assay method of the first example above in which the liposomes are adhered to the analyte DNA through the binding of complementary DNA sequences and the analyte is in turn adhered to the surface probe and so the sensor surface through the binding of complementary DNA sequences. Double stranded DNA can also be measured by the method of the second example. Double stranded DNA can also be related qualitatively and quantitatively to the presence of a specific target nucleic acid within a DNA amplification reaction (such as PCR or any isothermal amplification method). This enables sensitive detection of a target nucleic acid.

The target nucleic acid might itself be a label in an assay for a further analyte. For example, the target nucleic acid might be a label of an antibody or other specific recognition element which specifically binds a further analyte and so detection of the target nucleic acid may enable highly sensitive detection of a further analyte.

### Preparation of DNA samples

A specific set of primers (HR1F-HR1 R, Vorkas et al., Mutation scanning of exon 20 of the BRCA1 gene by high-resolution melting curve analysis. Clin. Biochem. 43 (2010) 178-185) was used to produce a 157bp DNA fragment from human genomic DNA. PCR reactions were set up following a PCR kit manufacturer's protocol (Kapa Biosystems, Wilmington, USA) in a final volume of 25 ml. 20 ng of human genomic DNA (Clontech Laboratories, Mountain View, USA) was used as template. The amplification protocol was set up as follows: 1 min initial denaturation at 95°C, 35 cycles consisted of 10 s denaturation at 95°C, 10 s annealing at 60°C, 10 s extension at 72°C, and 1 min of final extension at 72°C. 5 pmol of each of the two primers were added per reaction. The HR1F primer was biotinylated at its 5'-end. The HR1R was modified at its 5'end with cholesterol. PCR products were used either without post-PCR purification or after being purified using the Nucleospin PCR clean-up kit according to the manufacturer's instructions.

### Preparation of label

Liposomes were prepared having diameters D1, D2 and D3 of 50, 100 and 200 nm, respectively. A mixture of 2mg of lipids comprising 1-palmitoyl-2-oleoyl-sn-glycero-3-phosphocholine (POPC) was diluted in chloroform and kept at -20°C. The chloroform was evaporated with nitrogen and the lipids were left under a flow of nitrogen for an hour. The lipids were resuspended in a 1 ml of a buffer of 10 mM Tris, 200 mM NaCl, at a pH of 7.5 and vortexed for one hour. They were then extruded at least 21 times through a membrane chosen in dependence on the desired diameter. The resulting liposomes were then refrigerated until they were required and then dilute at least 10-fold in running buffer when used for experiments.

Note that apart from POPC, one skilled in the art will appreciate that a plethora of other commercially available lipids can also be used such as eg phosphatidyl choline (PC), dipalmitoylphosphatidylcholine (DPPC), 1,2-dioleoyl-sn-glycero-3-phosphatidylcholine (DOPC) etc, available for example from Avanti Polar Lipids, Inc. (Alabaster, USA) or other synthetic lipids such as NTA-lipids, lipids with PEG etc. Also, other molecules such as cholesterol, sterols etc. can also be incorporated in the liposomes structure, as well as lipids modified to carry a long tail at one end.

### Preparation of acoustic wave device

### QCM-D setup

Acoustic experiments were performed using the Q-Sense E4 instrument (QSense, Sweden). The latter includes four sensors that can be used in a parallel configuration. Prior to any experimental measurements one or more gold crystals were etched for 2.30 min at high power with a Harrick Plasma Cleaner using air. The gold sensors were immediately transferred to their chambers and filled with PBS buffer using a peristaltic pump. 200 ml of neutravidin (200 mg/ml) were loaded on the sensor under a constant flow of approximately 75 ml/min followed by PBS rinsing. QCM devices operating at 35 MHz were used to record the dissipation (D) and frequency (F) of the wave during the surface binding events.

### Measurements and Results

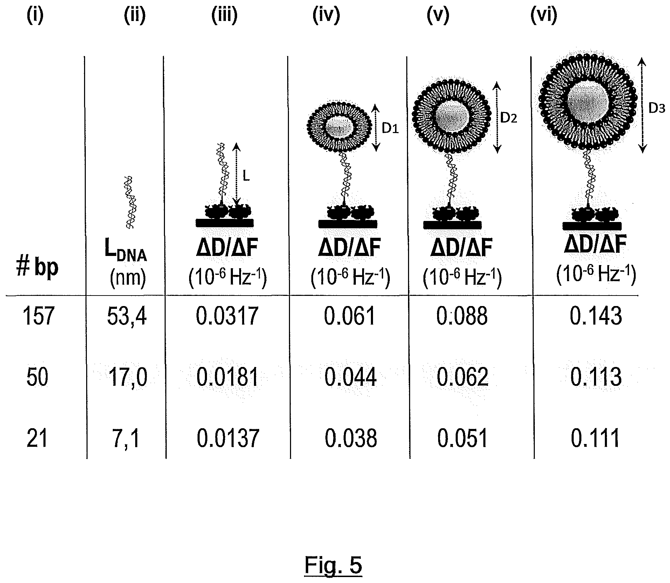

The table in Figure 5 shows in column (i) the length in base pairs and in column (ii) the length in nm, of the three lengths of dsDNA which were prepared. Column (iii) show the dissipative capacity (expressed as ΔD/ΔF) of double stranded DNA molecules, modified at a first end with biotin to bind to streptavidin on the sensor surface and at the other end with cholesterol to bind to liposomes, binding to the sensor surface. Columns (iv), (v) and (vi) show the measured dissipative capacity of liposomes of three diameters (iv) 50nm (D1), (v) 100nm (D2) and (vi) 200nm (D3) specifically binding to the tethered DNA molecules. Energy dissipation per unit mass (i.e., ΔD/ΔF) is expressed in units of 10⁻⁶ Hz⁻¹ and was obtained with a QCM device at 35 MHz.). In each case, the DNA/liposome surface coverage was low. Each result derived from a minimum of 10 experiments with a variation of 10-15%.

The table of Figure 5 clearly shows that the ratio ΔD/ΔF (dissipative capacity) is several times higher when liposomes bind to DNA then when the DNA molecules bind to the surface. The results show that dissipative capacity, ΔD/ΔF, is higher for longer DNA strands and for larger liposome diameters. Hence, the geometry of the attached entity (DNA length and liposome diameter) can be tuned to give higher or lower dissipation as preferred to optimise an assay.

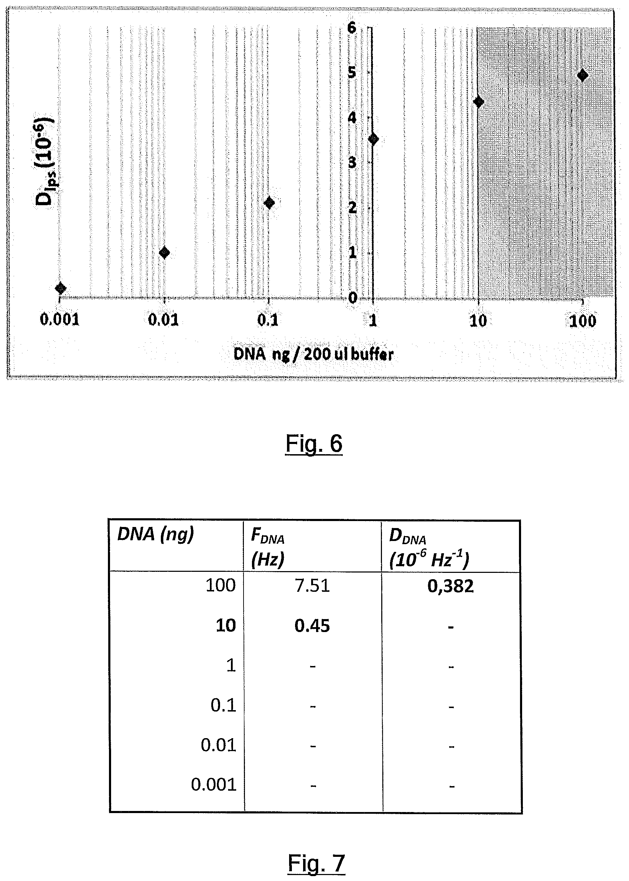

Figure 6 demonstrates the effect of using liposomes as a label to improve the detection limit of the DNA molecules. Using corresponding apparatus and protocols, the biotin and cholesterol modified 157bp dsDNA was added to the sensor surface in a range of amounts (0.001, 0.01, 0.1, 1, 10 and 100 ng in 200 microliters of buffer) and then the 200nm diameter liposome sample was applied. The change in dissipation, ΔD, before and after binding of the liposomes, was measured. Each data point is the average of 2 or 3 experiments.

The graph in Figure 6 demonstrates that this resulted in the detection of as low as 1pg of dsDNA (10 amoles or 6 million molecules) or an equivalent of 200 µl of 50 femtomolar DNA. In contrast, with reference to the table in Figure 6, it was only possible to detect a minimum of 10ng of the dsDNA by measuring the change in dissipation, ΔD, when the dsDNA sample was applied.

This new methodology showed that it is possible to detect acoustically 3 to 4 orders of magnitude lower DNA than that detected directly during the binding of the nucleic acid. Of interest is that his was achieved by just adding liposomes with only a 4 times higher dissipative capacity than DNA (in the top row of Figure 5, ΔD/ΔF was 0.143 when the largest size of liposome was added versus 0.0317 when the 157bp DNA was added, the ratio of the two is 0.143/.0317=4.5 but the detection limit improved by 3 to 4 orders of magnitude).

The remarkable improvement in DNA detection lies in the fact that the current invention exploits differences in the hydrodynamic properties of the two molecules: liposomes, being large and soft spheres, dissipate a significantly higher amount of energy as they oscillate when compared to the energy dissipated by more stiff DNA molecules.

A quantitative measure of the amount of analyte can be determined by comparing the parameter related to energy losses (e.g. ΔD, change in dissipation) or the parameter related to frequency (e.g. ΔF) or phase with a calibration curve.

### Use of an anchor

We have also found that assay sensitivity is improved when there is a spacer of between 5 and 250nm between the sensor surface and a body which makes up the bulk of the label, so that the label body is anchored 5 to 250nm from the sensor surface. Thus, where the analyte is a single stranded or double stranded nucleic acid and the DNA surface probe has a length of at least 15 base pairs, sensitivity is improved. Still further improvements are found for at least 29 base pairs (10nm) or at least 844 base pairs (15nm) or at least 59 base pairs (20nm). It is notable that in these experiments the dissipative capacity of 200nm liposomes (functioning as the body) increased by a factor of 1.3 as the surface bound double stranded DNA increased from 21 base pairs (7nm) to 157bp (53nm).

Thus in some embodiments according to the first example, the region 4 between the surface and the analyte capture region (shown in Figure 3A, 3B, 3C) can function as an anchor with a length of typically 10 nucleotides or more (provided that the length of the surface probe and analyte as a whole is at least 15 nucleotides to provide suitable spacing).

As well as being between the sensor surface and the analyte, the anchor may be part of the label, connecting a body (such as a liposome) to the analyte. Again, the anchor would typically have a length of between 5 and 250nm. Accordingly, liposomes 24 can be considered as the body and single stranded molecules 26 considered as the anchor.

Still further, the analyte (e.g. double stranded DNA) may itself function as an anchor having a length of between 5 and 250nm (Figure 4).

### Variations

The invention is applicable to a wide range of analytes as well as single or double stranded nucleic acids (DNA and RNA). For example, protein biomarkers such as proteins, glycoproteins, peptides, etc. and, low molecular weight analytes such as hormones, glucose, cholesterol, etc. and other metabolites. Specific binding of the analyte to the surface and the label to the analyte can be achieved using any appropriate specific recognition element, such as antibodies, antibody fragments, aptamers, chemical binding agents such as click chemistry or other types of specific recognition element known to those skilled in the art.

One skilled in the art will be aware of various chemistries which can be used to bind specific recognition elements to the sensor surface. For example, the DNA surface probe may instead be 5'-thiol modified to adhere to gold or another suitable sensor surface.

In order that the measurement is specific, the analyte typically binds specifically to the sensing surface and the label binds specifically to the analyte. However, it is not essential that both stages are specific, for example, it may be sufficient to specifically bind a target nucleic acid to the surface but then to use a label modified with a non-specific nucleic acid binding moiety.

Furthermore, additional specific recognition elements, such as antibodies or other specific binding molecules such as aptamers and affimers, may adhere the label to the analyte or adhere the label to the sensing surface (a sandwich assay format). In other embodiments, the label binds first to the analyte, before the analyte is bound to the sensing surface.

By way of example, in an alternative embodiment, once the analyte has bound to the surface probe DNA, a 5'-cholesterol-modified single stranded oligonucleic acid which has a sequence which is complementary to the second region (label capture region) of the analyte is introduced to the sensor surface in hybridizing conditions and binds to the analyte, where present. The liposomes may then be introduced and will adhere to the cholesterol moiety of the 5'-cholesterol-modified single stranded oligonucleic acid, thereby adhering the liposome label to the sensing surface in an amount corresponding to the amount of analyte which is bound.

In the examples given herein, liposomes were used as the highly dissipative label. However, other structures may be employed as label provided that they have an acoustic dissipative capacity which is significant higher than that of the target analyte. Examples of other highly dissipative structures which could be used as labels including liposomes of various sizes and compositions; beads or colloidal particles (of sufficiently dissipative material); vesicles consisting of non-lipid frameworks, such as polymers, dendrimers, and amphiphilic nanoparticles; cross-linked liposomes or vesicles; liposomes/vesicles employing floppy polymeric structures at the outside of the membrane; vesicles/liposomes encapsulating high viscous media instead of buffer; synthetic complexes where a central carrier (dendrimer) is used to bind two or three liposomes; polymerized lipid/polydiacetylene vesicles comprising lipids and polydiacetylene (PDA); and bolaamphiphile vesicles.

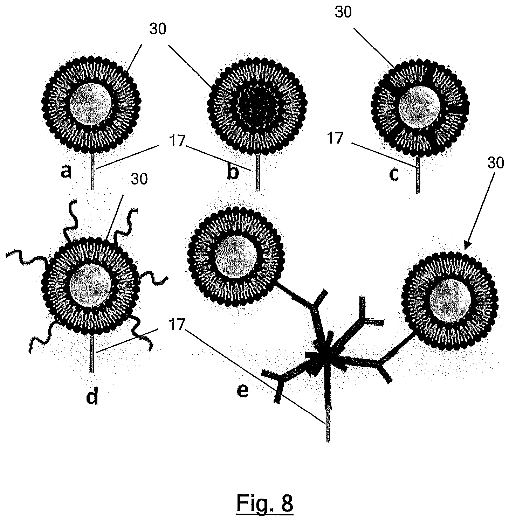

For example, Figure 8 illustrates some possible structures of label having a body 30 and spacer region 17 extending to a specific recognition element. In 8(a) the body is a liposome with a DNA probe which is complementary to a target nucleic acid sequence, as used in the present example. In 8(b) the body of the label a corresponding liposome which encapsulates a solution which is more viscous than the surrounding liquid. In 8(c) the body is a liposome corresponding to the example of Figure 8(a) except that some of the lipids have been cross-linked. In 8(d) illustrates liposomes with loss hydrophilic polymers (e.g. polyethylene glycol chains) extending into solution. 8(e) is an example in which the spacer region is a dendrimer complexed with multiple liposomes as the body. The spacer 17 can be a nucleic acid sequence, polymer such as polyethylene glycol etc.

The invention has considerable advantages. It enables highly specific detection of extremely low amounts of analytes including but not limited to DNA. It is therefore useful as an alternative to PCR for the detection of DNA and has various advantages including: lack of, or reduced requirements for DNA extraction; decreased time and cost; avoidance of PCR-bias risk; a requirement for relatively simple associated instrumentation with fewer heating elements and less power consumption. The acoustic system can be conveniently combined with microfluidics as described in Mitsakakis et al. K. Mitsakakis, et al, Journal of Microelectromechanical Systems 2008, 17, 1010-1019. Finally, the inherent simplicity and fully quantitative character of the proposed assay, together with the high sensitivity and ability of acoustic devices to integrate with other modules can be real assets for on-site analysis and low cost-operation, both crucial in applications such as personalized medicine and diagnostic platforms for the developing countries.

The invention may also be employed to detect an analyte after a limited number of rounds or a faster protocol of target analyte amplification (e.g. PCR or isothermal amplification). This may improve the detection limit but in comparison to using only PCR or isothermal amplification, which requires a large number (typically 30 to 50) of rounds of amplification, the overall detection process can be speeded up and PCR-bias minimised.

Hence, the assay is especially helpful for detection of analytes found in very low concentrations, for example for the detection of tumour circulating DNA (ctDNA) in blood.

Further modifications and variations may be made within the scope of the invention herein disclosed, as defined by the appended claims.

## Claims

1. A method of measuring an analyte (12) using a liquid medium acoustic wave sensor (1) having a sensing surface (4) and being an acoustic wave sensor which supports an acoustic wave that can propagate when the sensing surface of the acoustic wave sensor is in contact with a liquid, having a sensing surface (4), the method comprising adhering an analyte in a sample to the sensing surface through a single attachment point and adhering a label (14, 16, 18, 30) to the analyte and the surface such that the label binds discretely to the sensing surface, so that said label is adhered to the sensing surface through a single analyte, the label comprising a label body (14) which is thereby anchored to the sensing surface with an anchor length of 5-250nm, making a first measurement of the amplitude or dissipation of an acoustic wave generated by the liquid medium acoustic wave sensor before the label adheres to the surface; and making a second measurement of the amplitude or dissipation after the label adheres to the surface; and determining either or both the presence and amount of analyte from the change in the said amplitude or dissipation between the said first and second measurements, wherein the label has a dissipative capacity, being the ratio of the change in the energy losses of an acoustic wave generated by the liquid medium acoustic wave sensor to the change in the frequency or phase of the acoustic wave generated by the liquid medium acoustic wave sensor, due to the binding of the analyte or label to the sensing surface, which is at least 10% greater than that of the analyte.

2. A method according to claim 1, wherein the analyte (12) adheres to the surface (4) after the first measurement is made but before the second measurement is made and the method comprises making a preliminary measurement before the analyte adheres to the surface and then making the first measurement after the analyte adheres to the surface; and/or wherein the liquid medium acoustic wave sensor (1) is a bulk acoustic wave type device, or a surface acoustic wave device.

3. A method according to claim 1, wherein analyte (12) is adhered specifically to the sensing surface before the first measurement is taken.

4. A method according to claim 3 wherein following specific adherence of analyte to the sensing surface the label (14, 16, 18, 30) is then added and binds to the analyte (12), where present.

5. A method according to any one preceding claim, wherein a specific recognition element (18) is bound to the label body, the specific recognition element binding specifically to the analyte (12), and wherein optionally the specific recognition element (18) is bound to the label body (14) through a label bound spacer region (17, 19), the label bound spacer region having a length of at least 5nm.

6. A method according to claim 5, wherein a label bound probe (16) is bound to the label body, the label bound probe comprising a nucleic acid, the nucleic acid comprising the specific recognition element (18) and a spacer region (17, 19) which is intermediate the label body and the specific recognition element, the spacer region having a length of at least 15 nucleotides.

7. A method according to claim 5 or claim 6, wherein the label body (14) has a nucleic acid adhered thereto, the nucleic acid comprising a specific binding region (18) through which the analyte (12) adheres to the label body by hybridisation between the analyte and the specific binding region of the nucleic acid probe, and a spacer region (17, 19) intermediate the specific binding region and the label body, the nucleic acid having a length of 15 to 735 nucleotides, and the spacer region having a length of at least 15 nucleotides.

8. A method according to any one preceding claim, wherein a specific recognition element (6) is bound to the sensing surface (4), the specific recognition element binding specifically to the analyte (12), optionally wherein the specific recognition element (6) is bound to the sensing surface (4) through a surface bound spacer region (11, 13), the surface bound spacer region having a length of at least 5nm.

9. A method according to claim 8, wherein a surface probe (6) is bound to the sensing surface (4), the surface probe comprising a nucleic acid, the nucleic acid comprising a specific recognition (10) element and a spacer region (11, 13) which is intermediate the specific recognition element and the surface, the spacer region having a length of at least 15 nucleotides.

10. A method according to any one preceding claim where the analyte has a length, through which the label (14, 16, 18, 30) adheres to the sensing surface, of 5 - 250nm, wherein optionally the analyte (12) is double stranded DNA.

11. A method according to any one preceding claim, wherein the surface (4) has a nucleic acid (16) adhered thereto, the nucleic acid comprising a specific binding region (10) through which the analyte (12) and label (14, 16, 18, 30) adheres to the surface by hybridisation between the analyte and the specific binding region of the nucleic acid, and a spacer region (11, 13) intermediate the specific binding region and the sensing surface, the nucleic acid having a length of 15 to 735 nucleotides, and the spacer region having a length of at least 15 nucleotides.

12. A method according to any one preceding claim, wherein the label (14, 16, 18, 30) comprises liposomes (14, 30), wherein optionally the said liposomes (14, 30) encapsulate a medium with a viscosity of greater than 1 x 10⁻² Pa.s, and as a further option the label (14, 16, 18, 30) comprises group of a plurality of liposomes (30) which are joined to each other.

13. A method of selecting a label for the detection of an analyte (12) by a liquid medium acoustic wave sensor (1) having a sensing surface (4) and being an acoustic wave sensor which supports an acoustic wave that can propagate when the sensing surface of the acoustic wave sensor is in contact with a liquid, the method comprising measuring the change in energy losses of an acoustic wave generated by the liquid medium acoustic wave sensor (1) when the analyte binds to the sensing surface (4) of the liquid medium acoustic wave sensor, the analyte binding discretely to the sensing surface through a single attachment point, to thereby determine the dissipative capacity of the analyte and measuring the change in energy losses of an acoustic wave generated by the liquid medium acoustic wave sensor when a label (14, 16, 18, 30) binds to the surface, the label binding discretely to the sensing surface so that each label is adhered to the sensing surface through a single analyte, the label comprising a label body (14) which is thereby anchored to the sensing surface with an anchor length of 5-250nm, to thereby calculate the dissipative capacity of the label and selecting the label as a label for use in the detection of the analyte if the calculated dissipative capacity of the label is more than 10% greater than the calculated dissipative capacity of the analyte, the dissipative capacity being the ratio of the change in the energy losses of an acoustic wave generated by the liquid medium acoustic wave sensor to the change in the frequency or phase of the acoustic wave generated by the liquid medium acoustic wave sensor, due to the binding of the analyte or label to the sensing surface.

14. A method according to claim 13, wherein the analyte (12) is adhered to the sensing surface (4) through a spacer region having a length of 5 - 250nm; or wherein the label body is adhered to the analyte (12) through a spacer region having a length of 5 - 250nm; or wherein the analyte has a length, through which the label adheres to the surface, of 5 - 250nm, and optionally, the analyte (12) is double stranded DNA; and wherein as an additional option the surface has a nucleic acid adhered thereto, the nucleic acid comprising a specific binding region through which the analyte (12) and label (14, 16, 18, 30) adheres to the surface (4) by hybridisation between the analyte and the specific binding region of the nucleic acid probe, and a spacer region intermediate the specific binding region and the sensing surface, the nucleic acid having a length of 15 to 735 nucleotides, and the spacer region having a length of at least 10 nucleotides.

## Patentansprüche

1. Verfahren zum Messen eines Analyten (12) unter Verwendung eines Schallwellensensors (1) eines flüssigen Mediums, der eine Erfassungsoberfläche (4) aufweist und ein Schallwellensensor ist, der eine Schallwelle unterstützt, die sich ausbreiten kann, wenn die Erfassungsoberfläche des Schallwellensensors mit einer Flüssigkeit in Kontakt ist, der eine Erfassungsoberfläche (4) aufweist, das Verfahren umfassend ein Anhaften eines Analyten in einer Probe an der Erfassungsoberfläche über einen einzelnen Befestigungspunkt und das Anhaften einer Markierung (14, 16, 18, 30) an dem Analyten und der Oberfläche derart, dass die Markierung an die Erfassungsoberfläche diskret bindet, so dass die Markierung über einen einzelnen Analyten an der Erfassungsoberfläche anhaftet, die Markierung umfassend einen Markierungskörper (14), der dadurch an die Erfassungsoberfläche mit einer Ankerlänge von 5-250 nm verankert wird, wobei eine erste Messung der Amplitude oder der Dissipation einer Schallwelle durchgeführt wird, die durch den Schallwellensensor des flüssigen Mediums erzeugt wird, bevor die Markierung an der Oberfläche anhaftet; und Durchführen einer zweiten Messung der Amplitude oder der Dissipation, nachdem die Markierung an der Oberfläche anhaftet; und Bestimmen entweder des Vorhandenseins und der Menge des Analyten aus der Änderung der Amplitude oder der Dissipation zwischen der ersten und der zweiten Messung oder beides, wobei die Markierung eine dissipative Kapazität aufweist, die das Verhältnis der Änderung der Energieverluste einer Schallwelle, die durch den Schallwellensensor des flüssigen Mediums erzeugt wird, zu der Änderung der Frequenz oder der Phase der Schallwelle, die durch den Schallwellensensor des flüssigen Mediums erzeugt wird, aufgrund der Bindung des Analyten oder der Markierung an die Erfassungsoberfläche ist, die mindestens 10 % größer als die des Analyten ist.

2. Verfahren nach Anspruch 1, wobei der Analyt (12) an der Oberfläche (4) anhaftet, nachdem die erste Messung durchgeführt wird, jedoch bevor die zweite Messung durchgeführt wird, und das Verfahren das Durchführen einer vorläufigen Messung, bevor der Analyt an der Oberfläche anhaftet, und dann das Durchführen der ersten Messung umfasst, nachdem der Analyt an der Oberfläche anhaftet; und/oder
wobei der Schallwellensensor (1) des flüssigen Mediums eine Vorrichtung vom Volumenschallwellentyp oder eine Oberflächenschallwellenvorrichtung ist.

3. Verfahren nach Anspruch 1, wobei der Analyt (12) an der Erfassungsoberfläche spezifisch angehaftet wird, bevor die erste Messung vorgenommen wird.

4. Verfahren nach Anspruch 3, wobei, nach der spezifischen Anhaftung des Analyten an der Erfassungsoberfläche, die Markierung (14, 16, 18, 30) dann hinzugefügt wird und sich an den Analyten (12), sofern vorhanden, bindet.

5. Verfahren nach einem der vorstehenden Ansprüche, wobei ein spezifisches Erkennungselement (18) an den Markierungskörper gebunden ist, wobei das spezifische Erkennungselement an den Analyten (12) spezifisch bindet, und wobei optional das spezifische Erkennungselement (18) über einen markierungsgebundenen Abstandshalterbereich (17, 19) an den Markierungskörper (14) gebunden ist, wobei der markierungsgebundene Abstandshalterbereich eine Länge von mindestens 5 nm aufweist.

6. Verfahren nach Anspruch 5, wobei eine markierungsgebundene Sonde (16) an den Markierungskörper gebunden ist, die markierungsgebundene Sonde umfassend eine Nukleinsäure, die Nukleinsäure umfassend das spezifische Erkennungselement (18) und einen Abstandshalterbereich (17, 19), der zwischen dem Markierungskörper und dem spezifischen Erkennungselement liegt, wobei der Abstandshalterbereich eine Länge von mindestens 15 Nukleotiden aufweist.

7. Verfahren nach Anspruch 5 oder 6, wobei der Markierungskörper (14) eine Nukleinsäure daran anhaftend aufweist, die Nukleinsäure umfassend einen spezifischen Bindungsbereich (18), über den der Analyt (12) durch Hybridisierung zwischen dem Analyten und dem spezifischen Bindungsbereich der Nukleinsäuresonde an dem Markierungskörper anhaftet, und einen Abstandshalterbereich (17, 19), der zwischen dem spezifischen Bindungsbereich und dem Markierungskörper liegt, wobei die Nukleinsäure eine Länge von 15 bis 735 Nukleotiden aufweist und der Abstandshalterbereich eine Länge von mindestens 15 Nukleotiden aufweist.

8. Verfahren nach einem der vorstehenden Ansprüche, wobei ein spezifisches Erkennungselement (6) an die Erfassungsoberfläche (4) gebunden ist, wobei das spezifische Erkennungselement an den Analyten (12) spezifisch bindet, optional wobei das spezifische Erkennungselement (6) über einen oberflächengebundenen Abstandshalterbereich (11, 13) an die Erfassungsoberfläche (4) gebunden ist, wobei der oberflächengebundene Abstandshalterbereich eine Länge von mindestens 5 nm aufweist.

9. Verfahren nach Anspruch 8, wobei eine Oberflächensonde (6) an die Erfassungsoberfläche (4) gebunden ist, die Oberflächensonde umfassend eine Nukleinsäure, die Nukleinsäure umfassend ein spezifisches Erkennungs(10)-Element und einen Abstandshalterbereich (11, 13), der zwischen dem spezifischen Erkennungselement und der Oberfläche liegt, wobei der Abstandshalterbereich eine Länge von mindestens 15 Nukleotiden aufweist.

10. Verfahren nach einem der vorstehenden Ansprüche, wobei der Analyt eine Länge von 5-250 nm aufweist, über die die Markierung (14, 16, 18, 30) an der Erfassungsoberfläche anhaftet, optional wobei der Analyt (12) eine doppelsträngige DNA ist.

11. Verfahren nach einem der vorstehenden Ansprüche, wobei die Oberfläche (4) eine Nukleinsäure (16) daran anhaftend aufweist, die Nukleinsäure umfassend einen spezifischen Bindungsbereich (10), über den der Analyt (12) und die Markierung (14, 16, 18, 30) durch Hybridisierung zwischen dem Analyten und dem spezifischen Bindungsbereich der Nukleinsäure an der Oberfläche anhaften, und einen Abstandshalterbereich (11, 13), der zwischen dem spezifischen Bindungsbereich und der Erfassungsoberfläche liegt, wobei die Nukleinsäure eine Länge von 15 bis 735 Nukleotiden aufweist und der Abstandshalterbereich eine Länge von mindestens 15 Nukleotiden aufweist.

12. Verfahren nach einem der vorstehenden Ansprüche, wobei die Markierung (14, 16, 18, 30) Liposomen (14, 30) umfasst, wobei optional die Liposomen (14, 30) ein Medium mit einer Viskosität von mehr als 1 x 10⁻² Pa.s einkapseln, und als eine weitere Option die Markierung (14, 16, 18, 30) eine Gruppe aus einer Vielzahl von Liposomen (30) umfasst, die miteinander verbunden sind.

13. Verfahren zum Auswählen einer Markierung für die Feststellung eines Analyten (12) durch einen Schallwellensensor (1) des flüssigen Mediums, der eine Erfassungsoberfläche (4) aufweist und ein Schallwellensensor ist, der eine Schallwelle unterstützt, die sich ausbreiten kann, wenn die Erfassungsoberfläche des Schallwellensensors in Kontakt mit einer Flüssigkeit ist, das Verfahren umfassend das Messen der Änderung der Energieverluste einer Schallwelle, die durch den Schallwellensensor (1) des flüssigen Mediums erzeugt werden, wenn der Analyt an die Erfassungsoberfläche (4) des Schallwellensensors des flüssigen Mediums bindet, wobei der Analyt über einen einzigen Befestigungspunkt an die Erfassungsoberfläche diskret bindet, um dadurch die dissipative Kapazität des Analyten zu bestimmen, und das Messen der Änderung der Energieverluste einer Schallwelle, die durch den Schallwellensensor des flüssigen Mediums erzeugt wird , wenn eine Markierung (14, 16, 18, 30) an die Oberfläche bindet, wobei die Markierung an die Erfassungsoberfläche diskret bindet, so dass jede Markierung über einen einzigen Analyten an der Erfassungsoberfläche anhaftet, die Markierung umfassend einen Markierungskörper (14), der dadurch an der Erfassungsoberfläche mit einer Ankerlänge von 5-250 nm verankert wird, um dadurch die dissipative Kapazität der Markierung zu berechnen und die Markierung als eine Markierung zur Verwendung bei der Feststellung des Analyten auszuwählen, falls die berechnete dissipative Kapazität der Markierung mehr als 10 % größer als die berechnete dissipative Kapazität des Analyten ist, wobei die dissipative Kapazität das Verhältnis der Änderung der Energieverluste einer Schallwelle, die durch den Schallwellensensor des flüssigen Mediums erzeugt wird, zu der Änderung der Frequenz oder der Phase der Schallwelle, die durch den Schallwellensensor des flüssigen Mediums erzeugt wird, aufgrund der Bindung des Analyten oder der Markierung an die Erfassungsoberfläche ist.

14. Verfahren nach Anspruch 13, wobei der Analyt (12) über einen Abstandshalterbereich, der eine Länge von 5-250 nm aufweist, an der Erfassungsoberfläche (4) anhaftet; oder wobei der Markierungskörper über einen Abstandshalterbereich, der eine Länge von 5-250 nm aufweist, an dem Analyten (12) anhaftet; oder wobei der Analyt eine Länge von 5-250 nm aufweist, über die die Markierung an der Oberfläche anhaftet, und optional, der Analyt (12) die doppelsträngige DNA ist; und wobei als eine zusätzliche Option, die Oberfläche eine Nukleinsäure daran anhaftend aufweist, die Nukleinsäure umfassend einen spezifischen Bindungsbereich, über den der Analyt (12) und die Markierung (14, 16, 18, 30) durch Hybridisierung zwischen dem Analyten und dem spezifischen Bindungsbereich der Nukleinsäuresonde an der Oberfläche (4) anhaften, und einen Abstandshalterbereich, die zwischen dem spezifischen Bindungsbereich und der Erfassungsoberfläche liegt, wobei die Nukleinsäure eine Länge von 15 bis 735 Nukleotiden aufweist und der Abstandshalterbereich eine Länge von mindestens 10 Nukleotiden aufweist.

## Revendications

1. Procédé de mesure d'un analyte (12) à l'aide d'un capteur d'ondes acoustiques en milieu liquide (1) ayant une surface de détection (4) et étant un capteur d'ondes acoustiques qui prend en charge une onde acoustique qui peut se propager lorsque la surface de détection du capteur d'ondes acoustiques est en contact avec un liquide, ayant une surface de détection (4), le procédé comprenant l'adhésion d'un analyte dans un échantillon à la surface de détection par un seul point d'attache et l'adhésion d'un marqueur (14, 16, 18, 30) à l'analyte et à la surface de telle sorte que le marqueur se lie de manière discrète à la surface de détection, de sorte que ledit marqueur adhère à la surface de détection par un seul analyte, le marqueur comprenant un corps de marqueur (14) qui est ainsi ancré à la surface de détection avec une longueur d'ancrage de 5 à 250 nm, la réalisation d'une première mesure de l'amplitude ou de la dissipation d'une onde acoustique générée par le capteur d'ondes acoustiques en milieu liquide avant l'adhésion du marqueur à la surface ; et la réalisation d'une seconde mesure de l'amplitude ou de la dissipation après l'adhésion du marqueur à la surface ; et la détermination de la présence ou de la quantité d'analyte, ou des deux, à partir de la variation de ladite amplitude ou dissipation entre lesdites première et seconde mesures, dans lequel le marqueur a une capacité de dissipation, qui est le rapport entre la variation des pertes d'énergie d'une onde acoustique générée par le capteur d'ondes acoustiques en milieu liquide et la variation de la fréquence ou de la phase de l'onde acoustique générée par le capteur d'ondes acoustiques en milieu liquide, en raison de la liaison de l'analyte ou du marqueur à la surface de détection, qui est supérieur d'au moins 10 % à celui de l'analyte.

2. Procédé selon la revendication 1, dans lequel l'analyte (12) adhère à la surface (4) après la réalisation de la première mesure, mais avant la réalisation de la seconde mesure, et le procédé comprend la réalisation d'une mesure préliminaire avant l'adhésion de l'analyte à la surface, puis la réalisation de la première mesure après l'adhésion de l'analyte à la surface ; et/ou
dans lequel le capteur d'ondes acoustiques en milieu liquide (1) est un dispositif du type à ondes acoustiques de masse, ou un dispositif à ondes acoustiques de surface.

3. Procédé selon la revendication 1, dans lequel l'analyte (12) adhère spécifiquement à la surface de détection avant que la première mesure ne soit faite.

4. Procédé selon la revendication 3 dans lequel, après l'adhésion spécifique de l'analyte à la surface de détection, le marqueur (14, 16, 18, 30) est ensuite ajouté et se lie à l'analyte (12), le cas échéant.

5. Procédé selon l'une quelconque revendication précédente, dans lequel un élément de reconnaissance spécifique (18) est lié au corps de marqueur, l'élément de reconnaissance spécifique se liant spécifiquement à l'analyte (12), et dans lequel, facultativement, l'élément de reconnaissance spécifique (18) est lié au corps de marqueur (14) par une région d'espacement (17, 19) liée au marqueur, la région d'espacement liée au marqueur ayant une longueur d'au moins 5 nm.

6. Procédé selon la revendication 5, dans lequel une sonde (16) liée au marqueur est liée au corps de marqueur, la sonde liée au marqueur comprenant un acide nucléique, l'acide nucléique comprenant l'élément de reconnaissance spécifique (18) et une région d'espacement (17, 19) qui se trouve entre le corps de marqueur et l'élément de reconnaissance spécifique, la région d'espacement ayant une longueur d'au moins 15 nucléotides.

7. Procédé selon la revendication 5 ou la revendication 6, dans lequel le corps de marqueur (14) a un acide nucléique qui y adhère, l'acide nucléique comprenant une région de liaison spécifique (18) à travers laquelle l'analyte (12) adhère au corps de marqueur par hybridation entre l'analyte et la région de liaison spécifique de la sonde d'acide nucléique, et une région d'espacement (17, 19) entre la région de liaison spécifique et le corps de marqueur, l'acide nucléique ayant une longueur de 15 à 735 nucléotides, et la région d'espacement ayant une longueur d'au moins 15 nucléotides.

8. Procédé selon l'une quelconque revendication précédente, dans lequel un élément de reconnaissance spécifique (6) est lié à la surface de détection (4), l'élément de reconnaissance spécifique se liant spécifiquement à l'analyte (12), facultativement dans lequel l'élément de reconnaissance spécifique (6) est lié à la surface de détection (4) par une région d'espacement (11, 13) liée à la surface, la région d'espacement liée à la surface ayant une longueur d'au moins 5 nm.

9. Procédé selon la revendication 8, dans lequel une sonde de surface (6) est liée à la surface de détection (4), la sonde de surface comprenant un acide nucléique, l'acide nucléique comprenant un élément de reconnaissance spécifique (10) et une région d'espacement (11, 13) qui se trouve entre l'élément de reconnaissance spécifique et la surface, la région d'espacement ayant une longueur d'au moins 15 nucléotides.

10. Procédé selon l'une quelconque revendication précédente, où l'analyte a une longueur, à travers laquelle le marqueur (14, 16, 18, 30) adhère à la surface de détection, de 5 à 250 nm, facultativement dans lequel l'analyte (12) est un ADN double brin.

11. Procédé selon l'une quelconque revendication précédente, dans lequel la surface (4) a un acide nucléique (16) qui y adhère, l'acide nucléique comprenant une région de liaison spécifique (10) à travers laquelle l'analyte (12) et le marqueur (14, 16, 18, 30) adhèrent à la surface par hybridation entre l'analyte et la région de liaison spécifique de l'acide nucléique, et une région d'espacement (11, 13) entre la région de liaison spécifique et la surface de détection, l'acide nucléique ayant une longueur de 15 à 735 nucléotides, et la région d'espacement ayant une longueur d'au moins 15 nucléotides.

12. Procédé selon l'une quelconque revendication précédente, dans lequel le marqueur (14, 16, 18, 30) comprend des liposomes (14, 30), dans lequel facultativement lesdits liposomes (14, 30) encapsulent un milieu ayant une viscosité supérieure à 1 x 10⁻² Pa.s, et comme option supplémentaire, le marqueur (14, 16, 18, 30) comprend un groupe d'une pluralité de liposomes (30) qui sont reliés les uns aux autres.

13. Procédé de sélection d'un marqueur pour la détection d'un analyte (12) par un capteur d'ondes acoustiques en milieu liquide (1) ayant une surface de détection (4) et étant un capteur d'ondes acoustiques qui prend en charge une onde acoustique qui peut se propager lorsque la surface de détection du capteur d'ondes acoustiques est en contact avec un liquide, le procédé comprenant la mesure de la variation de pertes d'énergie d'une onde acoustique générée par le capteur d'ondes acoustiques en milieu liquide (1) lorsque l'analyte se lie à la surface de détection (4) du capteur d'ondes acoustiques en milieu liquide, l'analyte se liant de manière discrète à la surface de détection par un seul point d'attache, pour ainsi déterminer la capacité de dissipation de l'analyte et la mesure de la variation de pertes d'énergie d'une onde acoustique générée par le capteur d'ondes acoustiques en milieu liquide lorsqu'un marqueur (14, 16, 18, 30) se lie à la surface, le marqueur se liant de manière discrète à la surface de détection de sorte que chaque marqueur adhère à la surface de détection par un seul analyte, le marqueur comprenant un corps de marqueur (14) qui est ainsi ancré à la surface de détection avec une longueur d'ancrage de 5 à 250 nm, pour ainsi calculer la capacité de dissipation du marqueur et la sélection du marqueur comme marqueur à utiliser pour la détection de l'analyte si la capacité de dissipation calculée du marqueur est supérieure de plus de 10 % à la capacité de dissipation calculée de l'analyte, la capacité de dissipation étant le rapport entre la variation des pertes d'énergie d'une onde acoustique générée par le capteur d'ondes acoustiques en milieu liquide et la variation de la fréquence ou de la phase de l'onde acoustique générée par le capteur d'ondes acoustiques en milieu liquide, en raison de la liaison de l'analyte ou du marqueur à la surface de détection.

14. Procédé selon la revendication 13, dans lequel l'analyte (12) adhère à la surface de détection (4) par une zone d'espacement ayant une longueur de 5 à 250 nm ; ou dans lequel le corps de marqueur est collé à l'analyte (12) par une région d'espacement ayant une longueur de 5 à 250 nm ; ou dans lequel l'analyte a une longueur, à travers laquelle le marqueur adhère à la surface, de 5 à 250 nm, et facultativement, l'analyte (12) est un ADN double brin ; et dans lequel, comme option supplémentaire, la surface a un acide nucléique qui y adhère, l'acide nucléique comprenant une région de liaison spécifique par laquelle l'analyte (12) et le marqueur (14, 16, 18, 30) adhèrent à la surface (4) par hybridation entre l'analyte et la région de liaison spécifique de la sonde d'acide nucléique, et une région d'espacement entre la région de liaison spécifique et la surface de détection, l'acide nucléique ayant une longueur de 15 à 735 nucléotides, et la région d'espacement ayant une longueur d'au moins 10 nucléotides.
